# EUROPEAN PATENT APPLICATION

(11) **EP 3 381 375 A1**
(43) Date of publication of application: **03.10.2018**
(21) Application number: 17163263.1
(22) Date of filing: 28.03.2017
(51) Int. Cl.: A61B 10/02, A61B 10/00

(54) **SAMPLER FOR TAKING A SAMPLE FROM A BODY CAVITY, FOR EXAMPLE A CERVICAL SAMPLE**

(71) Applicant: Hol, Pieter, 34513 Waldeck (DE)
(72) Inventor: Hol, Pieter, 34513 Waldeck (DE)
(74) Representative: Patentanwälte Walther Hinz Bayer PartGmbB

(57) **Abstract**

The subject matter of the invention is a sampler for taking a sample from a body cavity, for example as cervical sample, comprising a rigid tube (12) having an introduction end (18) to be introduced in said cavity, said tube (12) comprises storage means to store and carry the sample and at its introduction end (18) an opening (20) to allow the sample to pass through. To create a sampler for taking a sample from a body cavity which can be easily and at low costs filled with the flushing fluid in a steril envirement and is able to suck enough sample fluid to conduct a reliable examination is achieved by storage means comprising a squeezable bellow (16), whereas the bellow (16) is located inside the tube (12) and whereas said bellow (16) has a front end heading towards said opening and a back end opposite of the front end.

## Description

The present invention relates to a sampler for taking a sample from a body cavity, for example as cervical sample, according to the preamble of the patent claim 1.

To counteract the disastrous consequences of cervical cancer, screening progams have been developed worldwide in which women undergo widespread examination. The usual method is to carry out a cervical smear (pap smear) by trained medical stuff, especially by gynaecologists, as described for example in EP 0 947 164 A1. However, a lot of woman involved find this an unpleasant experience and in addition, it is inconvenient to have to visit the medical facility in question.

To overcome these inconveniences WO-A-2006/033 569 discloses a sampler having a rigid tube, storage means, as well as pump and vacuum means. The pump and vacuum means consist of a plunger located inside the tube, whereas the plunger is moved by a handle.

When delivered, this sampler carries in his storage means 15 - 50 ml of a flushing fluid. During usage the user pushes the plunger inside the tube, thereby flushing the fluid through an opening at the introduced end of the tube into the body cavity. Thereafter, the user pulles the plunger backwards or a spring urges the plunger backwards respectively, to create a vacuum inside the tube. This vacuum sucks the sample from the body cavity through the opening into the tube. It is understood that this sample carries the cervical smear to be examined.

To produce a sampler according to WO-A-2006/033 569 it is necessarry to provide a steril envirement when filling in the flushing fluid. Because of the complexity of the sampler this is expensive.

Another problem is, that sometimes the operator or the spring respectively does not pull the plunger entirley backwards with the result, that the storage is not completely filled with sample fluid, hence in some cases there is not enough sample fluid to conduct a reliable examination.

Consequently, the present invention is based on the objective to provide a sampler for taking a sample from a body cavity which can be easily and at low costs filled with the flushing fluid in a steril envirement. Another objective is to provide a sampler being able to suck enough sample fluid to conduct a reliable examination.

As a technical solution of said objectives a sampler for taking a sample from a body cavity, for example as cervical sample, is proposed having the features of claim 1. Advantageous further embodiments of said sampler can be taken from the dependent claims.

A sampler for taking a sample from a body cavity, for example as cervical sample, according to the present invention has the advantage that the tube and the bellow are two distinct pieces, whereas only the bellow has to be filled with the flushing fluid. Because the bellow has no movable parts it is fairly easy to fill in the fluid and seal the bellow under steril conditions in an industrial scale and thereafter insert the bellow into the tube of the sampler.

In a preferred embodiment, the bellow is made from a material having a memory function. This makes the bellow act like a spring, that means after pushing the plunger and therby squeezing the bellow while flushing the fluid out of the sampler, the bellow will move back itself due to the memory function of the material into its starting position. Thereby, the vaccum inside the bellow will suck the sample fluid inside the bellow.

This has the advantage that the bellow allways moves back into its starting position, assuming the user stays away from the plunger and thereby the bellow automatically sucks enough sample fluid including the cervical smear to enable a reliable examination.

In another preferred embodiment the sampler comprises means for retaining the bellow in a defined position inside the tube. This ensures, that the bellow stays in its working position near the opening of the tube during the sucking period to enable a proper intake of the sample fluid.
In yet another preferred embodiment, the means for retaining the bellow comprise a first rigid shoulder, a second rigid shoulder and a third flexible shoulder. The first rigid shoulder is attached to the inner side of the tube and the second rigid shoulder together with the third flexible shoulder is attached to the outside of the bellow or vice versa. Thereby, the second shoulder and the third shoulder are spaced apart from each other to host the first shoulder in between, once the bellow is in its working position. These shoulders will fix the bellow relativ to the tube in its working position. Another advantage are the low costs for providing these three shoulders.

In case the first and the third shoulder are positioned to retain the bellow in a delivery position, the plunger will push the bellow onto the first or third shoulder, respectively, and thereby fix the bellow in the delivery postion, this is even more advantageous. In case the first and the second shoulder are positioned to retain the bellow in a working position, this is even more advantegeous.

In another, especially preferred emodiment the third shoulder is made of a flexible material to allow the shoulder to move over the first rigid shoulder while the bellow is moved from ist delivery in its working position. Once the working position is reached, the flexible shoulder will flash back into ist original position and host the first rigid shoulder between the second rigid shoulder and the third flexible shoulder.

This has the advantage, that the retaining means retain the bellow in a delivery postion while the third flexible shoulder touches the first rigid shoulder due to the pushing of the plunger during transport and storage of the sampler and that by pushing the plunger a little bit harder moving the third flexible shoulder over the first rigid shoulder until the second rigid shoulder touches the first rigid shoulder, therby retaining the bellow in its working position.

In a further preferred embodiment, inside the tube a breaking seal opener is provided and arranged to break the seal while the bellow is moved from its delivery position into its working position. This has the advantage that the bellow carrying the flushing fluid is sealed until shortly before it is used. Another advantage is that by using the inside breaking seal opener, no person has to touch the steril bellow to open it before the sampler is inserted into the body cavity and thereby a contamination of the bellow is avoided. This leads to a high standard steril use of the sampler.

It is in the nature of a bellow that the bellow carries a lost space inside, even if the bellow is completely squeezed. After pushing the plunger and thereby squeezing the bellow completely, some flushing fluid will remain in this lost space. After releasing the plunger, the bellow will move back in its origin position thereby sucking sample fluide inside the bellow. This sample fluid will be mixed with the flushing fluid of the lost space which leads to a dilution of the sample fluid. There is the danger that this diluted sample fluid can not be examined reliable. In order to avoid such a lost space in a prefered embodiment of the sampler according to the present invention a filling element is provided inside the bellow. It has found that it is advantegeous to size the filling element to at least 75% (better more) of the lost space.

In case the filling element is floating inside the bellow, this is even more advantages, since then the filling element will not disturb the squeezing of the bellow.

Further advantages of the sampler according to the present invention result from the attached drawing and the embodiments described below.

Likewise, the features mentioned herein and the features explained in more detail hereinafter can be used according to the invention individually or in any combination with each other. It is understood that the embodiments mentioned are not to be a limited list, they are rather exemplary. It is shown in :
- Fig. 1: a perpective view on a first embodiment of a sampler according to the present invention;
- Fig. 2a: a partly shown sampler according to Fig. 1 in a sectional side view according to line II - II in Fig. 1, whereas the bellow is in a first position;
- Fig. 2b: a partly shown sampler according to Fig. 1 in a sectional side view according to line II - II in Fig. 1, whereas the bellow is in a second position;
- Fig. 2c: a partly shown sampler according to Fig. 1 in a sectional side view according to line II - II in Fig. 1, whereas the bellow is in a third position;
- Fig. 2d: a partly shown sampler according to Fig. 1 in a sectional side view according to line II - II in Fig. 1, whereas the bellow is in a fourth position;
- Fig. 3: a top view on the sampler according to Fig. 1;
- Fig. 4a: a partly shown second embodiment of a sampler according to the present invention in a sectional side view, whereas the bellow is in a first position;
- Fig. 4b: a partly shown sampler according Fig. 3a in a sectional side view, whereas the bellow is in a second position;
- Fig. 4c: a partly shown sampler according to the present invention in a sectional side view, whereas the bellow is in a third position;
- Fig. 4d: a partly shown sampler according to the present invention in a sectional side view, whereas the bellow is in a fourth position.

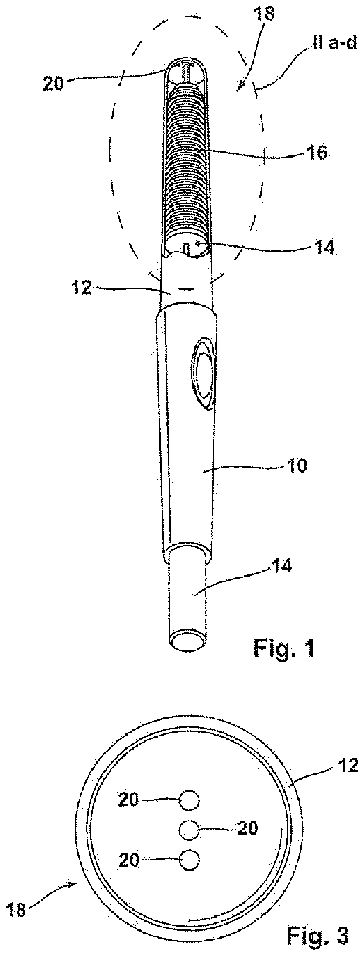

In the drawing Figs. 1 - 3 show a first embodiment of the sampler according to the present invention in order to carry out cervical smear from a body cavity to be examined in a laboratory to counteract cervical cancer. This sampler comprises a handling element 10 carrying a tube 12. At an open end of the handling element 10 a plunger 14 is inserted and inside the tube a bellow 16 is provided. The plunger 14 reaches through the handling element 10 into the tube 12 until it touches the bellow 16. The tube 12 is made of a rigid material and has an introduction end 18 to be inserted in a body cavity. At the tip of the introduction end 18 openings 20 are provided in the tube 12 to take in a sample fluid from the body cavity, as can be seen more detailed in Fig. 3. Details of the method to take the sample fluid inside are described below.

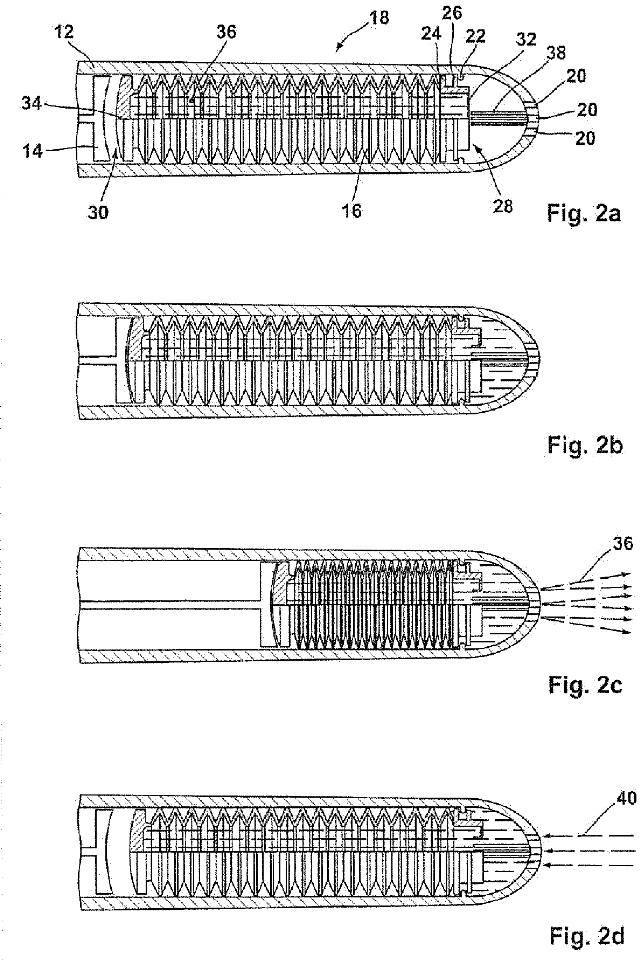

Figs. 2a -2d show a part of the sampler in a sectional view, whereas the bellow is shown in four different positions. As can be seen in, for example, Fig 2a more detailed, inside the tube 12 a circumferential first shoulder 22 made of a rigid material is provided, protruding rectangular into the inside of the tube 12. At the front end of the bellow 16 there is a second circumferential shoulder 24 made of a rigid material provided, also protruding rectangular inside the tube 12 and there is a third circumferential shoulder 26 made of a flexible material. The second shoulder 24 and the third shoulder 26 are spaced apart to host the first shoulder 22 in between.

The first 22, the second 24 and the third 26 shoulder are part of the means for retaining the bellow 16 in a defined postion inside the tube 12.

The bellow 16 has a front end 28 heading towards the openings 20 and a back end 30 just opposite of the front end 28. The regualarly open front end 28 is closed by a breaking seal 32, prefarably made of a membrane. In another embodiment the breaking seal also could be a kork or a paper lid. In the back end 30 a closable filling opening 34 is provided. Inside the bellow 16 a flushing fluid 36 is carried. In hence another embodiment, the back end is closed and the filling of the bellow will be conducted using the front end.

Inside the tube 12 adjacent to the openings 20 there is a breaking seal opener 38 reaching close to the front end 28 of the bellow 16 carrying the breaking seal 32 but not touching the breaking seal 32, while the bellow 16 is in its delivery postion as shown in Fig. 2a. In this delivery position the bellow 16 is pushed inside the tube 12 by the plunger 14 until the third shoulder 26 touches the first shoulder 22 and kept in this position during transport and storage of the sampler.

Once the sampler is to be used, the user inserts the sampler with its introduction end 18 into the body cavity as much as reasonable possible and then pushes the plunger 14 into the tube 12. Thereby the plunger 12 pushes forward the bellow 16 and the third flexible shoulder 26 is then bended so far that the third shoulder 26 can pass the first rigid shoulder 22. The second rigid shoulder 24 can not pass the first shoulder 22, but when the second rigid shoulder 24 touches the first rigid shoulder 22, the bellow 16 is in its working position as can be seen in Fig. 2b.

While moving the bellow 16 from its delivery position into its working position, the breaking seal opener 38 comes into effect with the breaking seal 32 and opens the breaking seal 32 before the bellow 16 reaches its working position as can be seen in Fig. 2b.

While pushing the plunger 14 even more, the bellow 16 will be squeezed and thereby the flushing fluid 36 from inside the bellow 16 will be injected via the broken breaking seal 32 of the front end 28 and the openings 20 of the tube 12 into the body cavity as can be seen in Fig. 2c. In the body cavity, the flushing fluid 36 eases cervical smear from the body.

Now, the user has to release the plunger 14 and at this moment, the material of the bellow 16 having the memory function urges the bellow 1 fi, and if necessarry the plunger 14, back into its starting postion and thereby creating a vaccuum inside the bellow 16. In this moment, the third shoulder 26 touches the first shoulder 22 and prevents the bellow 16 from moving backwards. Due to this vaccuum a sample fluid 40 from body cavity will be sucked into the bellow 16 via the openings 20 of the tube 12 and the front end 28 of the bellow 16, as can be seen in Fig. 2d. This sample fluid 40 is a mixture of the flushing fluid 36 together with the cervical smear from the body cavity. After the sucking is completed, the sample can be withdrawn from the body cavity. Thereafter, the sample fluid is to be delivered into a vial by pushing the plunger once more, Finally, the vial carrying the sample fluid 40 can be delivered to a laboratory in order to get the cervical smear examined in view of indications for cervical cancer.

It is understood that the third shoulder 26 is made of a flexible material suitable to allow the third shoulder 26 to pass the first shoulder 22 while the bellow 16 is moved from its delivery position into its working position and at the same time is stiff enough to hold the bellow 16 in its working position while the sample fluid 40 is sucked into the bellow 16.

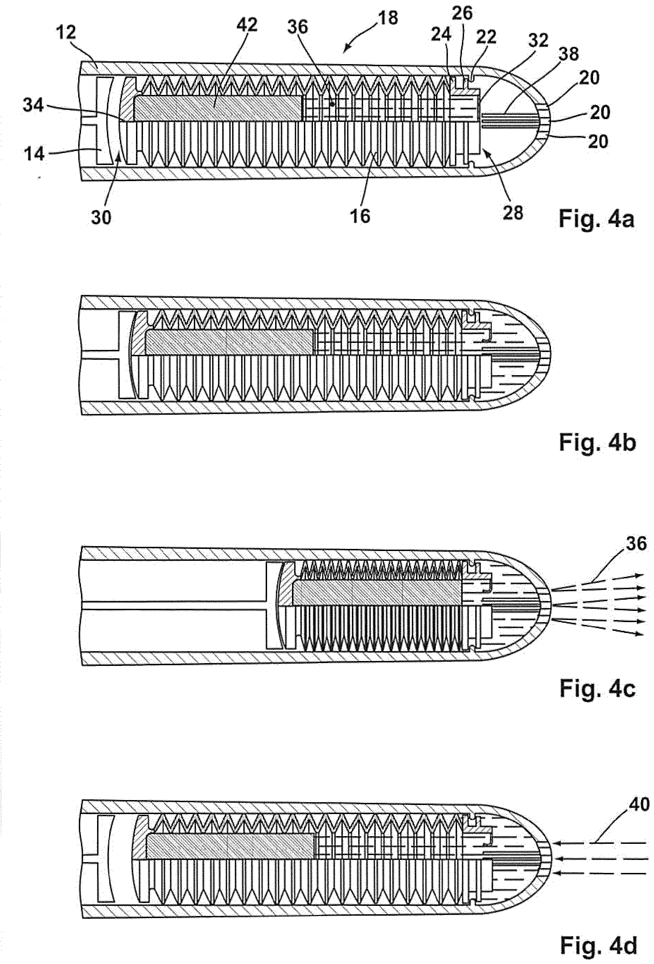

Figs. 4a - 4d show a second embodiment of the sampler according to the present invention. This second embodiment is identical with the first embodiment shown in Figs. 1 - 3 and additionally carries a filling element 42 inside the bellow 16. This filling element 42 fills up at least 75 % of the lost space when the bellow 16 is squeezed completely, as can be seen especially in Fig. 4c. Due to this filling element 42 the is less flushing fluid 36 inside the bellow 16 when delivered, but more important, there is a almost pure sample fluid 40 inside the bellow 16 in the end which makes an examination of the sample fluid much easier.

## Claims

1. Sampler for taking a sample from a body cavity, for example as cervical sample, comprising a rigid tube (12) having an introduction end (18) to be introduced in said cavity, said tube (12) comprises storage means to store and carry the sample and at its introduction end (18) an opening (20) to allow the sample to pass through,
**characterized in that**
the storage means comprise a squeezable bellow (16), whereas the bellow (16) is located inside the tube (12) and whereas said bellow (16) has a front end (28) heading towards said opening (20) and a back end (30) opposite of the front end (28).

2. Sampler according to claim 1,
**characterized in that**
said bellow (16) is made from a material having a memory function to enable the bellow (16) to act as a spring.

3. Sampler according to one of the preceding claims,
**characterized in that**
means for retaining the bellow (16) in a defined position inside the tube (12) are provided.

4. Sampler according to claim 3,
**characterized in that**
said means comprise a first shoulder (22) made of a rigid material provided at an inner side of the tube (12), a second shoulder (24) made of a rigid material provided at an outer side of the bellow (16), and a third shoulder (26) made of a flexible material provided at an outer side of the bellow (16), whereas the second (24) and the third shoulder (26) of the bellow (16) are spaced from each other to host the first shoulder (22) in between, once the bellow (16) is in its working position.

5. Sampler according to one of the preceding claims 3 or 4,
**characterized in that**
the first (22) and the third shoulder (26) are positioned in order to retain the bellow (16) in a delivery position.

6. Sampler according to one of the preceding claims 3 through 5,
**characterized in that**
the first (22) and the second shoulder (24) are positioned in order to retain the bellow (16) in a working position.

7. Sampler according to one of the preceding claims 3 through 6,
**characterized in that**
the third, flexible shoulder (26) is made of such a flexible material to enable the user to push the bellow (16) from ist delivery position into the working position and thereby moving the flexible shoulder (26) over the first, rigid shoulder (22) and to hold the bellow (16) in its working position during sucking the sample fluid (40) inside.

8. Sampler according to one of the preceding claims,
**characterized in that**
said bellow (16) comprises at its regularly open front end (28) a breaking seal (32), prefarably a membrane, to close the open front end (28).

9. Sampler according to claim 8,
**characterized in that**
a breaking seal opener (38) is provided inside the tube (12), preferably at the introduction end (18) of the tube (12).

10. sampler according to claim 9,
**characterized in that**
the breaking seal opener (38) is sized not to open the breaking seal (32) as long as the bellow (16) is in its delivery position and that the breaking seal opener (38) is sized to open the breaking seal (32) before the bellow (16) has reached its working position.

11. Sampler according to one of the preceding claims,
**characterized in that**
inside the bellow (16) a filling element (42) is provided.

12. Sampler according to claim 11,
**characterized in that**
the filling element (42) is sized to fill up at least 75 %, better more, of the space of the bellow (16) in its squeezed position.

13. Sampler according to one of the preceding claims 11 or 12,
**characterized in that**
the filling element (42) is floating inside the bellow (16).

14. Sampler according to one of the preceding claims,
**characterized in that**
said bellow (16) comprises a closable filling opening (34) at its back end (30).

## Amended claims

### Amended claims in accordance with Rule 137(2) EPC.

1. Sampler for taking a sample from a body cavity, for example as cervical sample, comprising a rigid tube (12) having an introduction end (18) to be introduced in said cavity, said tube (12) comprises storage means to store and carry the sample and at its introduction end (18) an opening (20) to allow the sample to pass through,
**characterized in that**
the storage means comprise a squeezable bellowss (16) said bellowss (16) is made from a material having a memory function to enable the bellows (16) to act as a spring, whereas the bellows (16) is located inside the tube (12) and whereas said bellows (16) has a front end (28) heading towards said opening (20) and a back end (30) opposite of the front end (28).

2. Sampler according to one of the preceding claims,
**characterized in that**
means for retaining the bellows (16) in a defined position inside the tube (12) are provided.

3. Sampler according to claim 2,
**characterized in that**
said means comprise a first shoulder (22) made of a rigid material provided at an inner side of the tube (12), a second shoulder (24) made of a rigid material provided at an outer side of the bellows (16), and a third shoulder (26) made of a flexible material provided at an outer side of the bellows (16), whereas the second (24) and the third shoulder (26) of the bellows (16) are spaced from each other to host the first shoulder (22) in between, once the bellows (16) is in its working position.

4. Sampler according to one of the preceding claim 3,
**characterized in that**
the first (22) and the third shoulder (26) are positioned in order to retain the bellows (16) in a delivery position.

5. Sampler according to one of the preceding claims 2 through 4,
**characterized in that**
the first (22) and the second shoulder (24) are positioned in order to retain the bellows (16) in a working position.

6. Sampler according to one of the preceding claims 2 through 5,
**characterized in that**
the third, flexible shoulder (26) is made of such a flexible material to enable the user to push the bellows (16) from ist delivery position into the working position and thereby moving the flexible shoulder (26) over the first, rigid shoulder (22) and to hold the bellows (16) in its working position during sucking the sample fluid (40) inside.

7. Sampler according to one of the preceding claims,
**characterized in that**
said bellows (16) comprises at its regularly open front end (28) a breaking seal (32), prefarably a membrane, to close the open front end (28).

8. Sampler according to claim 7,
**characterized in that**
a breaking seal opener (38) is provided inside the tube (12), preferably at the introduction end (18) of the tube (12).

9. Sampler according to claim 8,
**characterized in that**
the breaking seal opener (38) is sized not to open the breaking seal (32) as long as the bellows (16) is in its delivery position and that the breaking seal opener (38) is sized to open the breaking seal (32) before the bellows (16) has reached its working position.

10. Sampler according to one of the preceding claims,
**characterized in that**
inside the bellows (16) a filling element (42) is provided.

11. Sampler according to claim 10,
**characterized in that**
the filling element (42) is sized to fill up at least 75 %, better more, of the space of the bellows (16) in its squeezed position.

12. Sampler according to one of the preceding claims 10 or 11,
**characterized in that**
the filling element (42) is floating inside the bellows (16).

13. Sampler according to one of the preceding claims,
**characterized in that**
said bellows (16) comprises a closable filling opening (34) at its back end (30).
